# EUROPÄISCHE PATENTSCHRIFT

(11) **EP 1 840 120 B1**
(45) Veröffentlichungstag und Bekanntmachung des Hinweises auf die Patenterteilung: **24.04.2013**
(21) Anmeldenummer: 06006227.0
(22) Anmeldetag: 27.03.2006
(51) Int. Cl.: C07D 219/02, H01L 51/00, C09K 11/06, H05B 33/14, H05B 33/08

(54) **N-Heterozyklische Verbindungen und deren Verwendung in elektronischen, optoelektronischen und elektroluminiszenten Bauelementen.**
N-Heterocyclic compounds and their use in electronic, optoelectronic and electroluminiscent components
Composés N-Heterociclic et leur utilisation dans composants électroniques, optoélectroniques et électroluminiscents

(43) Veröffentlichungstag der Anmeldung: 03.10.2007
(73) Patentinhaber: Novaled AG, 01307 Dresden (DE)
(72) Erfinder: Grüssing, André, 64291 Darmstadt (DE); Lux, Andrea, 01277 Dresden (DE); Hartmann, Horst, 01326 Dresden (DE)
(74) Vertreter: Bittner, Thomas L.

(56) Entgegenhaltungen:
- WO-A-99/13691
- WO-A-2004/067675
- JP-A- 58 085 439
- JP-A- 2005 350 416
- US-A1- 2004 146 746
- PATENT ABSTRACTS OF JAPAN Bd. 2000, Nr. 26, 1. Juli 2002 (2002-07-01) & JP 2001 244076 A (TOYOTA CENTRAL RES & DEV LAB INC), 7. September 2001 (2001-09-07) & JP 2001 244076 A (TOYOTA CENTRAL RES & DEV LAB INC) 7. September 2001 (2001-09-07)

## Beschreibung

Die vorliegende Erfindung betrifft die Verwendung von N-Heterozyklischen Verbindungen in elektronischen, optoelektronischen und elektrolumineszenten Bauelementen, wie organischen lichtemittierenden Dioden (OLEDs), vorzugsweise als Ladungstransportmaterial oder Blockermaterial, besonders bevorzugt als Lochleiter oder Elektronenblocker.

In organischen lichtemittierenden Dioden (OLEDs) wird die Eigenschaft von Materialien ausgenutzt, Licht zu emittieren, wenn durch das Anlegen einer Spannung geeignete Ladungsträger gebildet werden, die bei ihrer Rekombination angeregte Zustände bilden, die unter Emission von Licht in den Grundzustand übergehen. OLEDs stellen eine interessante Alternative zu Kathodenstrahlröhren und Flüssigkristalldisplays dar, da sie aufgrund ihrer sehr kompakten Bauweise und ihres niedrigen Stromverbrauchs zur Herstellung von Flachbildschirmen und Displays für mobile Anwendungen wie Mobiltelefone, Notebooks, PDAs etc. geeignet sind.

Zur Verbesserung der Effizienz und Temperaturstabilität organischer lichtemittierender Dioden bestehen OLEDs häufig neben der eigentlichen Emissionsschicht aus Ladungstransportschichten, die für den Transport negativer und positiver Ladungsträger in die Emissionsschicht zuständig sind. Diese Ladungstransportschichten werden nach der Art der transportierten Ladungsträger in Lochleiter und Elektronenleiter eingeteilt.

Organische lichtemittierende Dioden (OLEDs) bestehen aus verschiedenen Schichten organischer Materialien, wobei mindestens eine Schicht (Emissionsschicht) eine elektrolumineszente Substanz enthält, die durch Anlegung einer Spannung zur Emission von Licht gebracht werden kann (C.W. Tang et al., Appl. Phys. Lett 51, 913 (1987).

Zur Verbesserung der Injektion der Ladungsträger in die Emissionsschicht und der Temperaturstabilität werden Ladungstransportschichten aus elektronenleitenden Materialien (Elektronenleiter) bzw. lochleitenden Materialien (Lochleiter) in die OLED integriert, die ebenfalls als Blockerschichten für die jeweiligen komplementären Ladungsträger fungieren können.

Dem Stand der Technik entsprechend werden als lochleitende bzw. elektronenblockende Materialien bisher Benzidin-Derivate wie etwa N,N'-Diphenyl-N,N'-bis(3-methylphenyl)-1,1'-biphenyl'-4,4'-diamin (TPD), 4,4'-Di-(N-carbazolyl)-diphenyl (CBP) und N,N'-Di-(alpha-naphthyl)-N,N'-diphenyl- 1,1'-biphenyl-4,4'-diamin (alpha-NPD) eingesetzt, 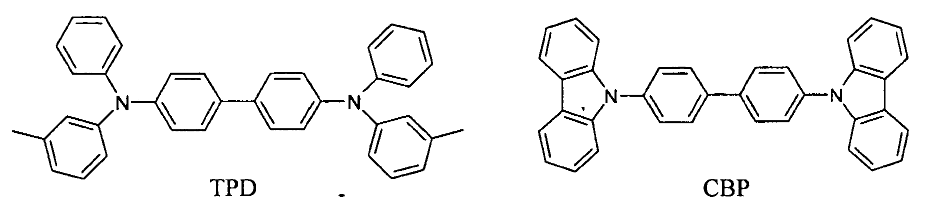 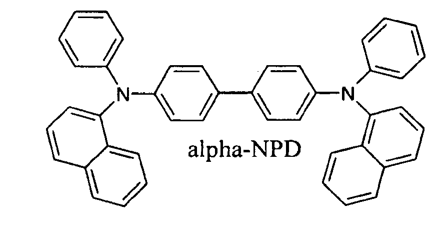 die jedoch eine mangelnde Temperaturstabilität aufweisen, die von der Glasübergangstemperatur dieser Verbindungen abhängt (TPD: T_{g}=65°C; CBP: kein Tg; alpha-NPD: T_{g}=105°C).

Die Verwendung von N-heterozyklischen Verbindungen als Ladungstransportmaterialien ist bisher auf geringfügig substituierte Acridan-Derivate und verwandte Verbindungen wie Carbazole beschränkt.

US 6,242,115 B1 betrifft OLEDs, in denen unsymmetrische Ladungstransportmaterialien eingesetzt werden, die tertiäre Amin-Funktionen beinhalten. Diese tertiären Amin-Funktionen sind aufgebaut aus dem Biphenyl-Kern und zwei zusätzlichen Phenylgruppen, die nicht miteinander verbunden, aber auch direkt oder über eine gesättigte oder ungesättigte Brücke miteinander verknüpft sein können. Als solche Verknüpfungen werden eine gesättigte bzw. ungesättigte C2-Brücke vorgeschlagen, die jedoch keine zusätzlichen Substituenten tragen.

US2004/0146746 betrifft eine blaue, elektrolumineszente Verbindung und ein organisches elektrolumineszentes Bauteil, in dem diese blaue, elektrolumineszente Verbindung Anwendung findet, wobei die Verbindung eine mit Alkoxy- oder Aminogruppen substituierte 9,10-Diphenylanthrazeneinheit umfasst.

W02004/067675 betrifft ein blaues, elektrolumineszentes Material, das auf einer Binaphthylverbindung basiert. Hier zeichnet sich die Binaphthylverbindung durch eine gute Löslichkeit in gebräuchlichen organischen Lösungsmitteln und eine hohe Widerstandsfähigkeit gegenüber Kristallisation aus. Die beschriebenen Verbindungen konnten zur Herstellung organischer elektrolumineszenter Bauteile eingesetzt werden.

WO99/13691 betrifft organische, lichtemittierende Bauteile, die organische Ladungstransportschichten aus einem Ladungstransportmaterial umfassen, das auf asymmetrisch substituierten aromatischen Verbindungen basiert. In diesen Verbindungen sind die lochtransportierenden Substituenten asymmetrisch um ein Atom oder eine chemische Gruppe herum angeordnet.

JP2005250416 betrifft eine Methode zur Herstellung von Arylamin hoher Reinheit bei geringen Kosten, die als elektronische Materialien, wie etwa positive Ladungstransportmaterialien in einem organischen Halbleiterelement, verwendet werden können. Insbesondere wird hier die Synthese von Triarylaminen oder Diarylaminen durch Reaktion aromatischer Amine mit aromatischen Halogenverbindungen unter Kupferkatalyse beschrieben.

WO 00/33671 betrifft lochleitende Materialien, in denen zwei tertiäre Aminfunktionen über eine Carbazol-Einheit verknüpft sind und Arylsubstituenten tragen, die wiederum nicht miteinander verbunden oder über eine ungesättigte C2-Brücke miteinander verbunden sein können. Diese Brücke kann wiederum unsubstituiert, substituiert oder Teil eines aromatischen Systems sein.

JP 10312073 A betrifft organische elektrolumineszente Bauelemente, deren lochleitende Schicht aus Starburst-Aminen mit unterschiedlichen Substituenten, darunter Acridan-Derivaten, besteht. -

JP 06293883 betrifft organische elektrolumineszente Bauelemente, die monomere oder dimere Acridan-Derivate enthalten. Die dimeren Acridan-Derivate sind direkt miteinander verknüpft.

EP 0764712 A2 betrifft elektrolumineszierende Anordnungen, die u.a. einen gegen Luft, Thermooxidation, Ozon und UV-Strahlung wirksamen Stabilisator enthalten. Als ein solcher Stabilisator werden auch Acridane vorgeschlagen.

JP 58085439 offenbart Verbindungen mit zwei substituierten Acridan-Einheiten.

JP 2001244076 beansprucht Acridan-Derivate in organischen, lumineszenten Bauelementen, jedoch keine diaryl-substituierten Verbindungen.

Bislang hat die mangelnde Temperaturstabilität der bisher in elektrolumineszenten Bauelementen eingesetzten lochleitenden bzw. elektronenblockenden Verbindungen einen technischen Mangel dargestellt, der die Anwendung dieser Bauelemente in temperatursensiblen Bereichen limitiert hat.

Es ist eine Aufgabe der vorliegenden Erfindung, neue, insbesondere lochleitende oder elektronenblockende, Verbindungen bereitzustellen, die die Nachteile des Stands der Technik überwinden und vorteilhaft in elektronischen, optoelektronischen und elektrolumineszenten Bauelementen, insbesondere in temperatursensiblen Bereichen, eingesetzt werden können. Auch ist es eine Aufgabe, Verbindungen bereitzustellen, die eine höhere Effizienz ermöglichen.

Diese Aufgabe wird erfindungsgemäß gelöst durch N-heterozyklische Verbindung gemäß der allgemeinen Formeln A oder B 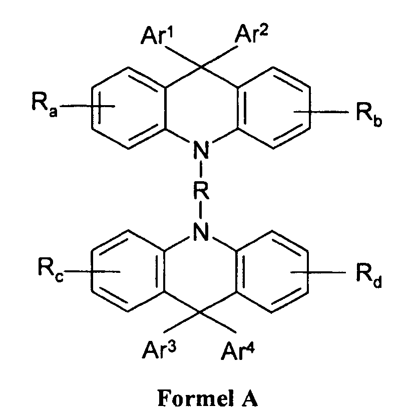 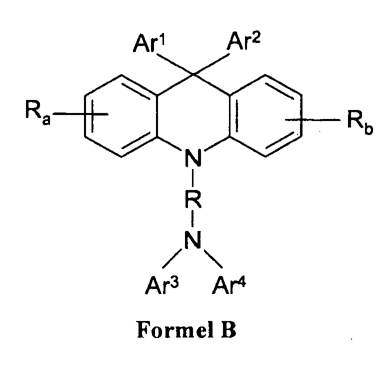 worin:
Ar¹, Ar², Ar³ und Ar⁴ unabhängig voneinander, substituiert oder unsubstituiert, beliebige aromatische oder heteroaromatische Reste sind;
Rₐ, R_{b}, R_{c} und R_{d} unabhängig voneinander Wasserstoff, Halogen und, substituiert oder unsubstituiert, Alkyl, Aryl in der für Ar¹-Ar⁴ genannten Bedeutung, anellierter Carbozyklus, anellierter Heterozyklus, Alkoxy oder Aryloxy sind, wobei Rₐ, R_{b}, R_{c} und R_{d} auch mehrfach auftreten können; und
R ein beide Stickstoff-haltige Gruppen verbrückendes, ein konjugationsfähiges System enthaltendes Strukturelement ist, wobei
R ausgewählt wird aus 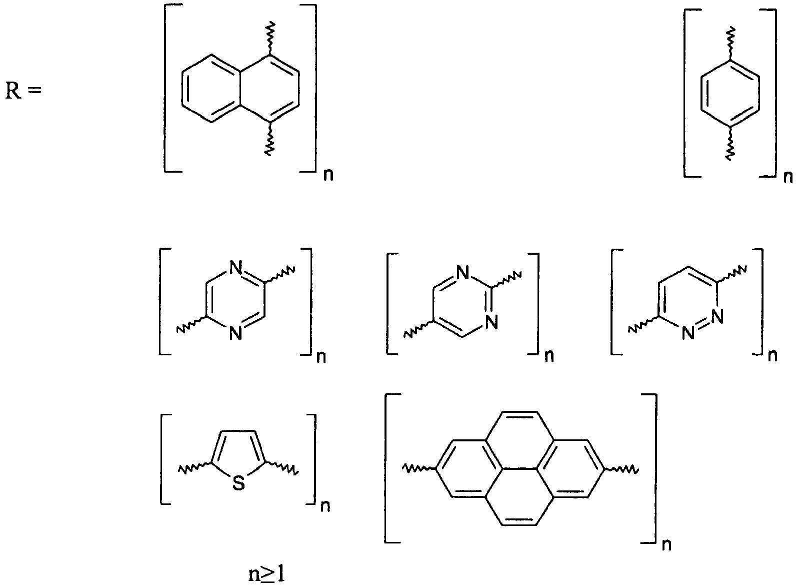 oder Biphenyl oder einen Dimeren, Trimeren oder Oligomeren eines solchen Strukturelements wobei Hydroxy-, Alkoxy-, Cycloalkoxy- und Aryloxy-substiuiertes Biphenyl ausgeschlossen ist, als Lochleiter in elektronischen, optoelektronischen und elektrolumineszenten Bauelementen.

Bevorzugt ist, dass Ar¹, Ar², Ar³ und Ar⁴ substituierte oder unsubstituierte carbozyklische aromatische Reste sind, vorzugsweise substituiertes oder unsubstituiertes Phenyl, 1-Naphthyl, 2-Naphthyl, 1-Anthryl, 2-Anthryl und 9-Anthryl.

Alternativ wird vorgeschlagen, dass Ar¹, Ar², Ar³ und Ar⁴ Homologe carbozyklischer aromatischer Reste sind, vorzugsweise beliebig verknüpftes und an die Acridan-Einheit gebundenes Biphenyl, Binaphthyl, Bianthryl und Pyrenyl.

Als weitere Alternative wird vorgesehen, dass Ar¹, Ar², Ar³ und Ar⁴ heteroaromatische Reste sind, vorzugsweise 2-Pyridyl, 3-Pyridyl, 4-Pyridyl, 2-Chinolyl, 3-Chinolyl, 4-Chinolyl, 5-Chinolyl , 6-Chinolyl , 7-Chinolyl, 8-Chinolyl, 2-Thienyl, 3-Thienyl und Thiazolyl.

Eine weitere alternative Ausführungsform sieht vor, dass Ar¹, Ar², Ar³ und Ar⁴ Homologe heteroaromatischer Reste sind, vorzugsweise beliebig verknüpftes und an die Acridan-Einheit gebundenes Bipyridyl, Bichinolyl und Bithienyl.

Schließlich wird ein elektronisches, optoelektronisches oder elektrolumineszentes Bauelement vorgeschlagen, das mindestens eine erfindungsgemäße Verbindung enthält.

Überraschenderweise wurde festgestellt, dass die offenbarten N-heterozyklischen Verbindungen eine hohe Temperaturstabilität aufweisen, wenn sie beispielsweise als lochleitende oder elektronenblockende Verbindungen in elektronischen, optoelektronischen oder elektrolumineszenten Bauelementen eingesetzt werden. Auch zeigen die erfindungsgemäßen Verbindungen eine sehr hohe Effizienz. Die erfindungsgemäß vorgeschlagenen N-heterozyklischen Verbindungen sind aus dem Stand der Technik bislang nicht bekannt, so dass auch deren Verwendungsmöglichkeiten nicht beschrieben worden sind.

In einer bevorzugten Ausführungsform der Erfindung können die Substituenten Ar¹, Ar², Ar³ und Ar⁴ die folgende, substituierte oder unsubstituierte Struktur aufweisen:

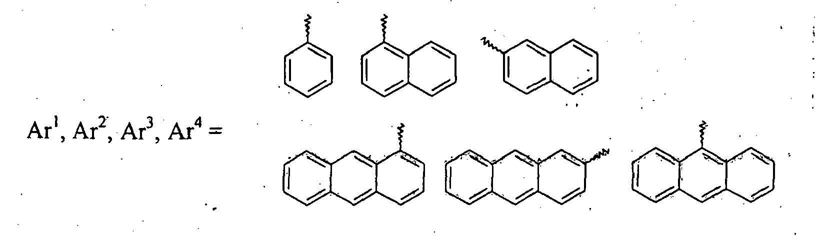

In einer weiteren bevorzugten Ausführungsform können die Substituenten Ar¹, Ar², Ar³ und Ar⁴ die folgenden, substituierten oder unsubstituierten Strukturen aufweisen:

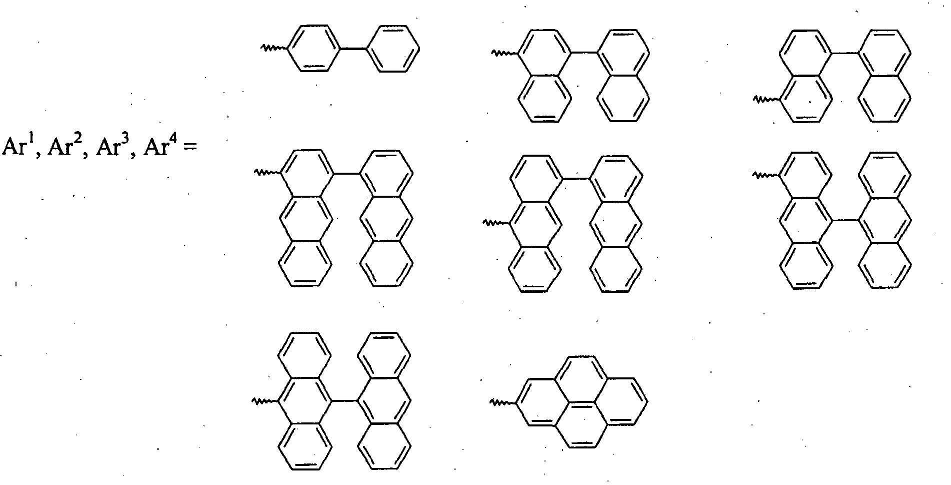

In einer noch weiter bevorzugten Ausführungsform der Erfindung können die Substituenten Ar¹, Ar², Ar³ und Ar⁴ die folgende, substituierte oder unsubstituierte Struktur aufweisen: 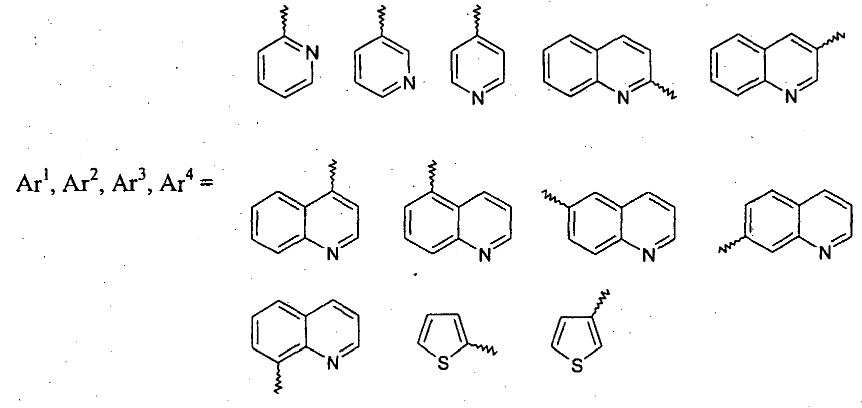 Schließlich können in einer anderen bevorzugten Ausführungsform der Erfindung die Substituenten Ar¹, Ar², Ar³ und Ar⁴ die folgende, substituierte oder unsubstituierte Struktur aufweisen:

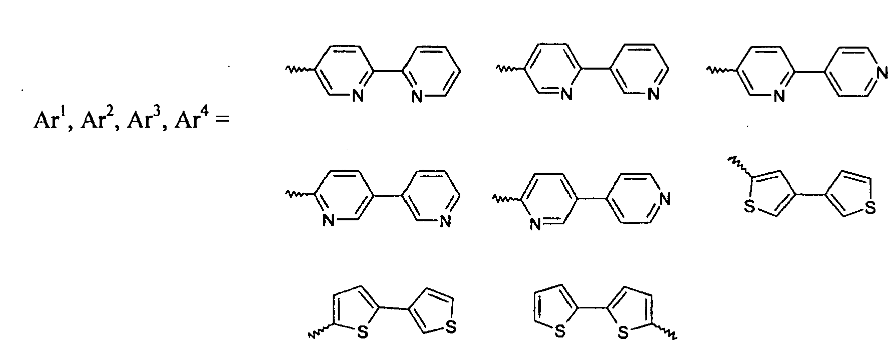

Geeignete Synthesewege, auf dem erfindungsgemäße Verbindungen zugänglich sind, sind für Verbindungen gemäß Formel A, 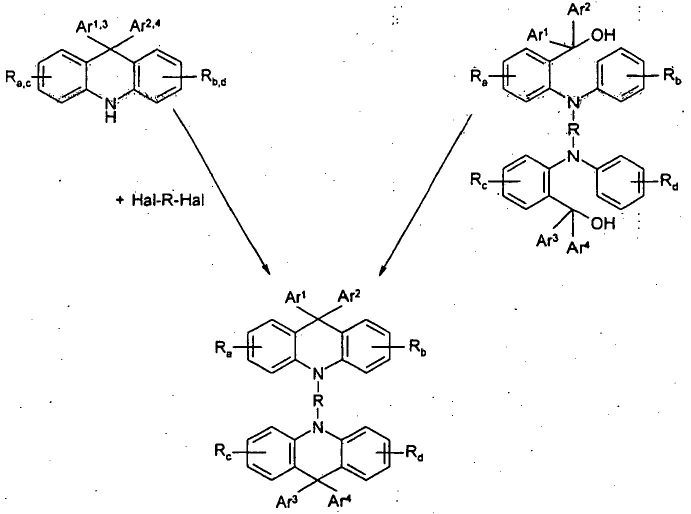 für Verbindungen gemäß Formel B, 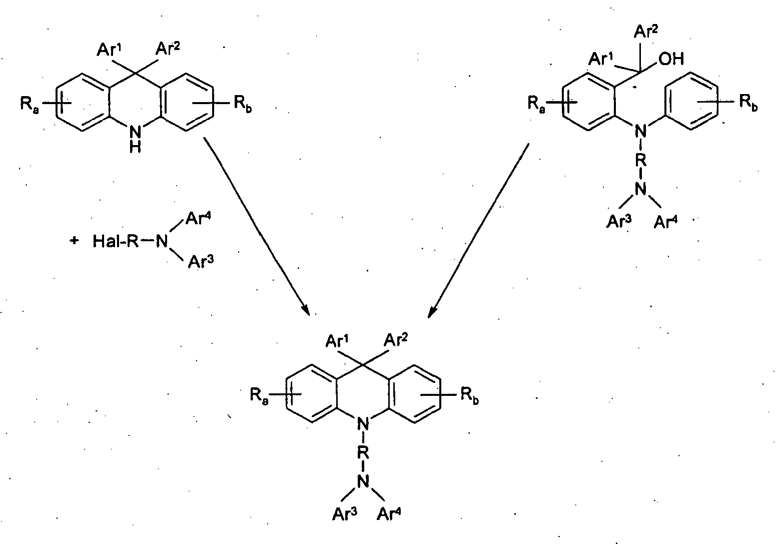 für Verbindungen gemäß Formel C, 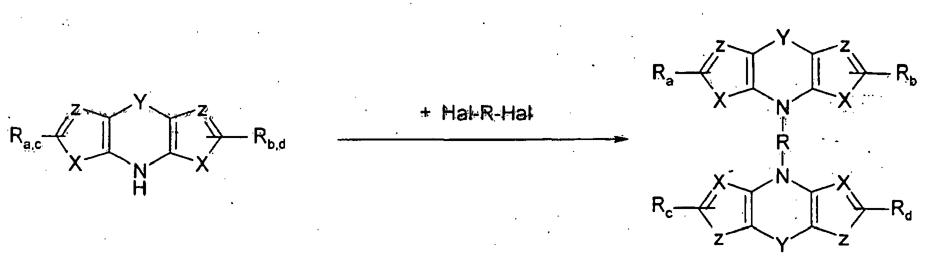 für Verbindungen gemäß Formel D, 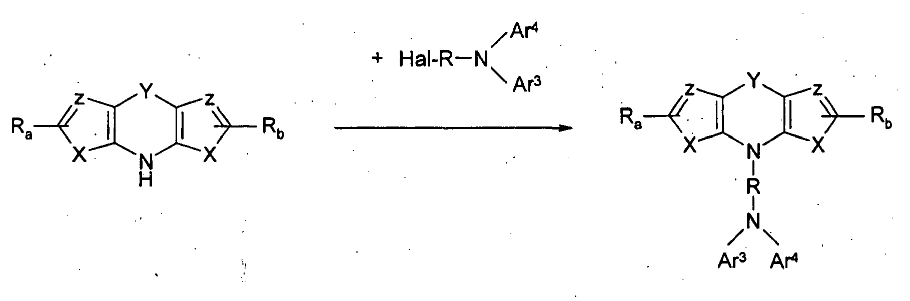 worin Ar¹-Ar⁴, X, Y, Z und R, Rₐ, R_{b}, R_{c}, R_{d} die vorstehend erläuterten Bedeutungen haben und Hal einen Halogensubstituenten bezeichnet. Geeignete Syntheseverfahren zur Herstellung der erfindungsgemäßen Verbindungen und ihrer Vorstufen sind dem Fachmann bekannt.

Weitere Merkmale und Vorteile der vorliegenden Erfindung ergeben sich aus der folgenden detaillierten Beschreibung einiger Ausführungsformen, die lediglich als beispielhaft zu betrachten sind und nicht so verstanden werden sollen, um den Umfang der Erfindung in irgendeiner Art und Weise zu begrenzen.

### Ausführungsbeispiel 1: 4,4'-Bi-(9,9-diphenyl-9,10-dihydroacridin)-biphenyl

Herstellung von N-Phenylanthranilsäuremethylester 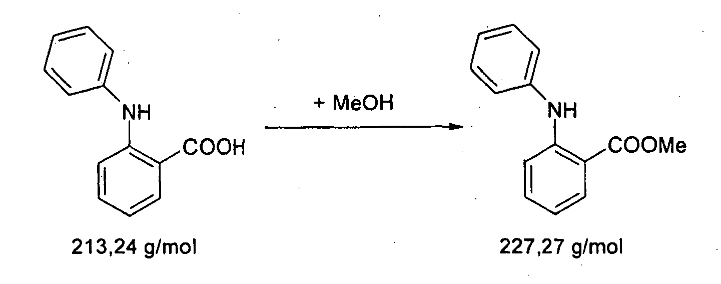 85,466 g N-Phenylanthranilsäure (400 mmol) wurden in 1 L MeOH suspendiert und die Suspension mit gasförmiger HCl gesättigt. Nach 7 Tagen bei RT wurde das Lösungsmittel entfernt, der Rückstand in 300 ml Toluol aufgenommen, filtriert und das Filtrat zur Trockne eingeengt. Das Rohprodukt wurde durch Destillation (1,1*10⁻¹ mbar, 117°C) gereinigt.
MS: m/z=227 [M]⁺

Herstellung von 9,9-Diphenyl-9,10-dihydroacridin

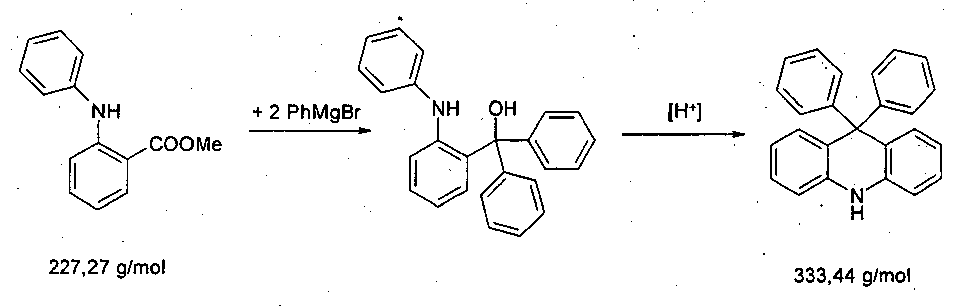

Die luft- und feuchtigkeitsempfindlichen Schritte der Synthese wurden mittels Schlenktechnik unter Argon in getrockneten Lösungsmitteln durchgeführt.

10,012 g N-Phenylanthranilsäuremethylester (44,05 mmol) wurden in 30 ml THF gelöst, bei -78°C mit 52 ml 2,8 M Phenylmagnesiumbromid in Et₂O (145,6 mmol) versetzt und 4 Tage bei RT gerührt. Nach Zugabe von 200 ml MeOH wurde das THF am Rotationsverdampfer entfernt und weitere 300 ml MeOH zugegeben. Die resultierende Suspension wurde mit konzentrierter HCl versetzt, worauf das Produkt als weißer Feststoff ausfiel, der filtriert und getrocknet wurde.
MS: m/z=333 [M]⁺, ¹H-NMR (500 MHz, DMSO-d₆):): 9,08 (s, 1H); 7,26-7,23 (t, 4H), 7,23-7,19 (t, 2H), 7,18-7,12 (t, 2H), 6,92-6,90 (d, 2H), 6,84-6,82 (d, 4H), 6,82-6,7 (t, 2H), 6,64-6,62 (d, 2H)

Herstellung von 4,4'-Bi-(9,9-diphenyl-9,10-dihydroacridin)-biphenyl

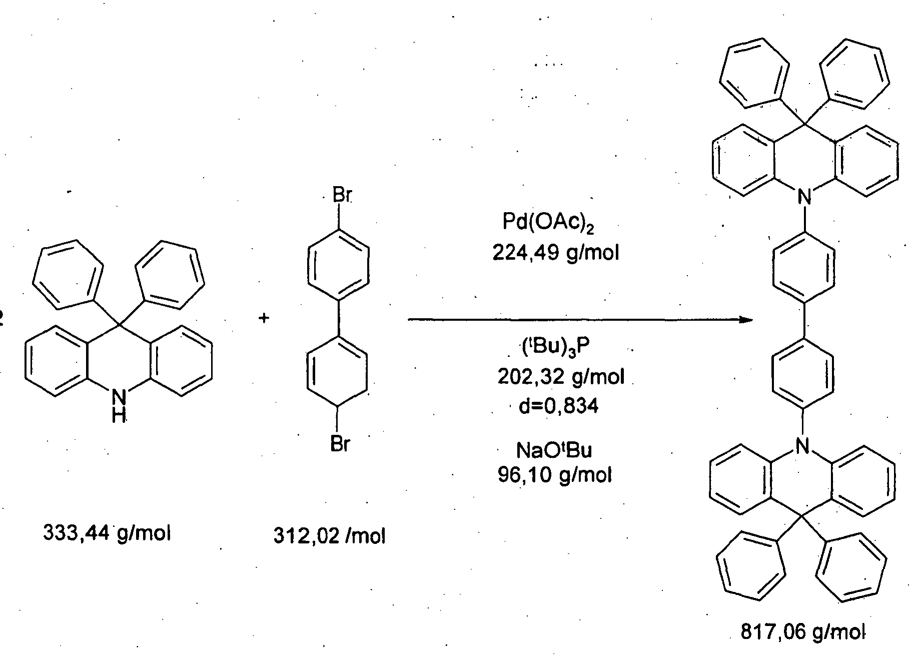

Die luft- und feuchtigkeitsempfindlichen Schritte der Synthese wurden mittels Schlenktechnik unter Argon in getrockneten Lösungsmitteln durchgeführt.

1,998 g 9,9-Diphenyl-9,10-dihydroacridin (6,0 mmol), 0,915 g 4,4'-Dibrombiphenyl (2,93 mmol), 0,725 g NaO^{t}Bu (7,54 mmol), 0,138 g Pd(OAc)₂ (0,6147 mmol) und 0,6 ml (^{t}Bu)₃P (2,47 mmol) wurden in 60 ml Toluol unter Rückfluss zum Sieden erhitzt. Bei RT wurden 100 ml CH₂Cl₂ und 60 ml Wasser zugegeben; die organische Phase abgetrennt, das Lösungsmittel vollständig entfernt, der Rückstand in 100 ml MeOH für 10 min unter Rückfluss zum Sieden erhitzt, filtriert und in 200 ml CH₂Cl₂ aufgenommen. Die Lösung wurde auf 50 ml eingeengt, filtriert, das Produkt mit MeOH und Et₂O gewaschen und getrocknet.
MS: m/z=739 [M-Ph]⁺, ¹H-NMR (500 MHz, DMSO-d₆): 8,0 (d, 4H), 7,35-7,30 (t, 8H), 7,30-7,27 (t, 4H), 7,19-7,17 (d, 4H), 7,13-7,10 (t, 4H), 6,93-6,91 (m, 12H), 6,79-6,78 (d, 4H), 6,45-6,43 (d, 4H)

### Ausführungsbeispiel 2: 1,4-Bi-(9,9-diphenyl-9,10-dihydroacridin)-phenyl

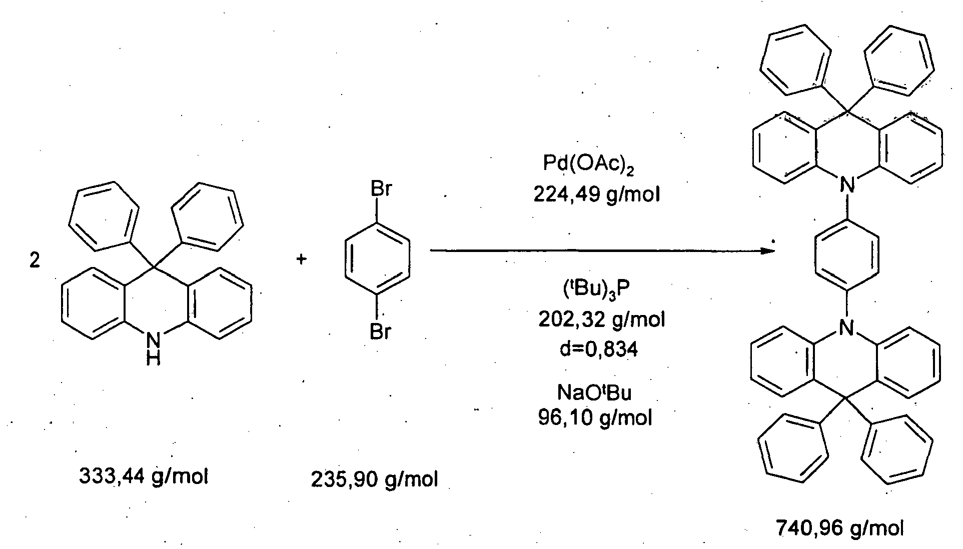

Die luft- und feuchtigkeitsempfindlichen Schritte der Synthese wurden mittels Schlenktechnik unter Argon in getrockneten Lösungsmitteln durchgeführt.

1,999 g 9,9-Diphenyl-9,10-dihydroacridin (6,0 mmol), 0,692 g 1,4-Dibromphenyl (2,93 mmol), 0,728 g NAO^{t}Bu (7,57 mmol), 0,140 g Pd(OAc)₂ (0,623 mmol) und 0,6 ml (^{t}Bu)₃P (2,47 mmol) wurden in 60 ml Toluol unter Rückfluss zum Sieden erhitzt. Bei RT wurden 300 ml CHCl₃ und 100 ml Wasser zugegeben, die organische Phase abgetrennt, mit 100 ml Wasser gewaschen, das Lösungsmittel vollständig entfernt, der Rückstand in 200 ml MeOH für 45 min unter Rückfluss zum Sieden erhitzt, filtriert und in 200 ml CH₂Cl₂ aufgenommen. Die Lösung wurde auf 50 ml eingeengt, filtriert, das Produkt mit MeOH und Et₂O gewaschen und getrocknet.
MS: m/z=740 [M]⁺; 663 [M-Ph]⁺, ¹H-NMR (500 MHz, DMSO-d₆): 7,29-7,21 (m, 12H), 7,19 (s, 4H), 7,12-7,09 (m, 4H), 6,99-6,97 (dd, 8H), 6,90 (d, 8H), 6,53-6,51 (d, 4H)

### Ausführungsbeispiel 3: 1-(9,9-diphenyl-9,10-dihydroacridin)-4-(diphenylamino)-phenyl

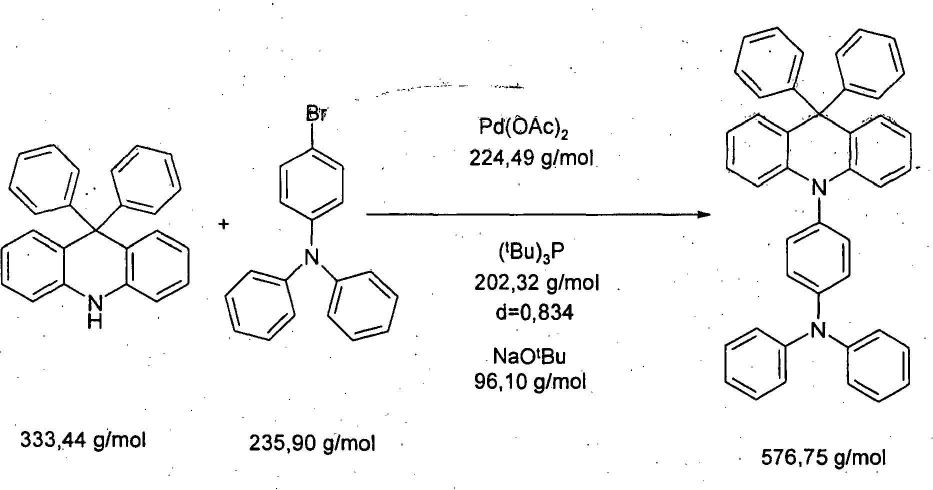

Die luft- und feuchtigkeitsempfindlichen Schritte der Synthese wurden mittels Schlenktechnik unter Argon in getrockneten Lösungsmitteln durchgeführt.

2,0 g 9,9-Diphenyl-9,10-dihydroacridin (6,0 mmol), 2,0 g 4-Bromphenyl-diphenylamin (6,1 mmol), 0,7 g NaO^{t}Bu (7,28 mmol), 0,140 g Pd(OAc)₂ (0,623 mmol) und 0,6 ml (^{t}Bu)₃P (2,47 mmol) wurden in 100 ml Toluol unter Rückfluss zum Sieden erhitzt. Bei RT wurden 200 ml CHCl₃ und 100 ml Wasser zugegeben, die organische Phase abgetrennt, mit 200 ml Wasser gewaschen, das Lösungsmittel vollständig entfernt, der Rückstand in 200 ml MeOH für 60 min unter Rückfluss zum Sieden erhitzt, filtriert, in Et₂O suspendiert, 2 min erhitzt, filtriert und getrocknet.
MS: m/z=576 [M]⁺, 499 [M-Ph]⁺, ¹H-NMR (500 MHz, CDCl₃): 7,30-7,26 (t, 4H), 7,24-7,20 (t, 6H), 7,16-7,14 (m, 6H), 7,10-7,03 (m, 4H), 6,98-6,96 (m, 4H), 6,87-6,85 (m, 6H), 6,55 (d, 2H)

Die in der vorstehenden Beschreibung und den Ansprüchen offenbarten Merkmale können sowohl einzeln als auch in jeder beliebigen Kombination für die Verwirklichung der Erfindung in ihren unterschiedlichen Ausführungsformen wesentlich sein.

## Patentansprüche

1. Verwendung einer N-heterozyklischen Verbindung gemäß den allgemeinen Formeln A oder B 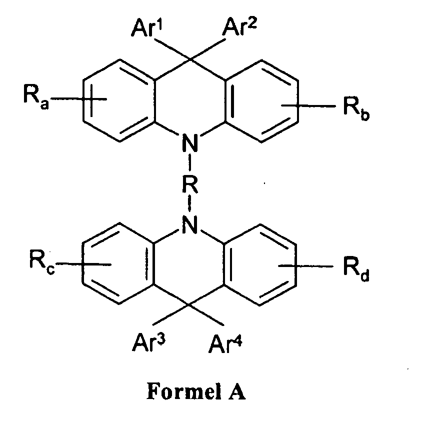 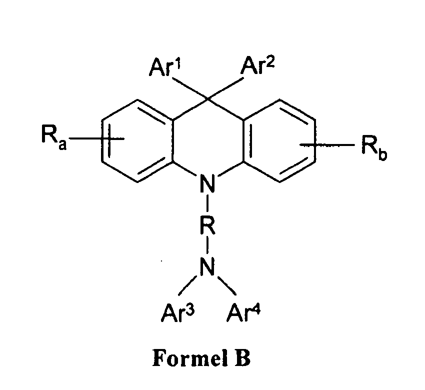 worin:
Ar¹, Ar², Ar³ und Ar⁴ unabhängig voneinander, substituiert oder unsubstituiert, beliebige aromatische oder heteroaromatische Reste sind;
Rₐ, R_{b}, R_{c} und R_{d} unabhängig voneinander Wasserstoff, Halogen, Alkyl, Aryl in der für Ar¹-Ar⁴ genannten Bedeutung, anellierter Carbozyklus, anellierter Heterozyklus, Alkoxy oder Aryloxy sind, wobei Rₐ, R_{b}, R_{c} und R_{d} auch mehrfach auftreten können; und
R ein beide Stickstoff-haltige Gruppen verbrückendes, ein konjugationsfähiges System enthaltendes Strukturelement ist, wobei R ausgewählt wird aus 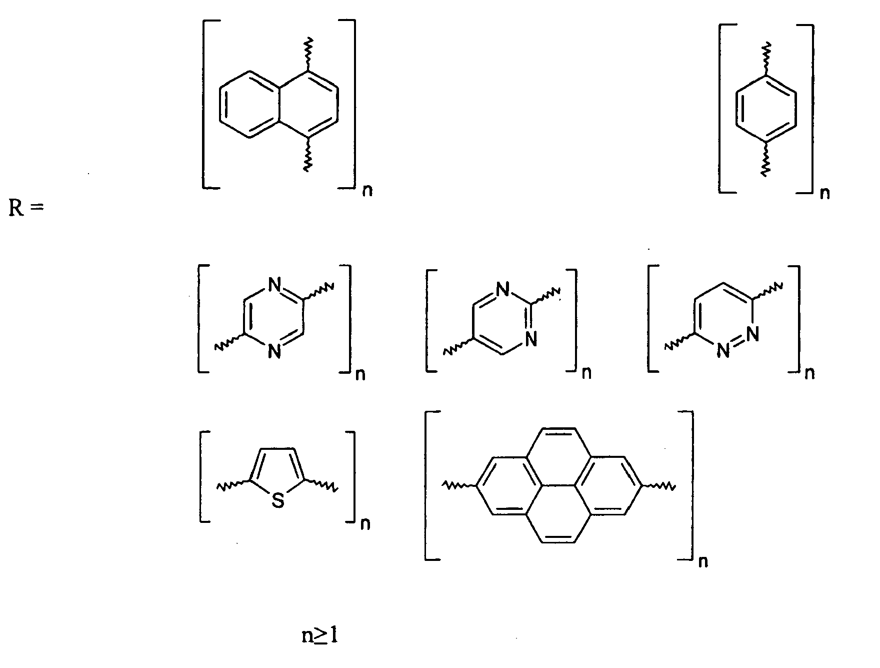 oder Biphenyl oder einen Dimeren, Trimeren oder Oligomeren eines solchen Strukturelements, wobei Hydroxy-, Alkoxy-, Cycloalkoxy- und Aryloxy-substituiertes Biphenyl ausgeschlossen ist, als Lochleiter in elektronischen, optoelektronischen und elektrolumineszenten Bauelementen.

2. Verwendung nach Anspruch 1, **dadurch gekennzeichnet, dass** Ar¹, Ar², Ar³ und Ar⁴ carbozyklische aromatische Reste sind, vorzugsweise Phenyl, 1-Naphthyl, 2-Naphthyl, 1-Anthryl, 2-Anthryl und 9-Anthryl.

3. Verwendung nach Anspruch 1, **dadurch gekennzeichnet, dass** Ar¹, Ar², Ar³ und Ar⁴ Homologe carbozyklischer aromatischer Reste sind, vorzugsweise beliebig verknüpftes und an die Acridan-Einheit gebundenes Biphenyl, Binaphthyl, Bianthryl und Pyrenyl.

4. Verwendung nach Anspruch 1, **dadurch gekennzeichnet, dass** Ar¹, Ar², Ar³ und Ar⁴ heteroaromatische Reste sind, vorzugsweise 2-Pyridyl, 3-Pyridyl, 4-Pyridyl, 2-Chinolyl, 3-Chinolyl, 4-Chinolyl, 5-Chinolyl , 6-Chinolyl , 7-Chinolyl, 8-Chinolyl, 2-Thienyl, 3-Thienyl und Thiazolyl.

5. Verwendung nach Anspruch 1, **dadurch gekennzeichnet, dass** Ar¹, Ar², Ar³ und Ar⁴ Homologe heteroaromatischer Reste sind, vorzugsweise beliebig verknüpftes und an die Acridan-Einheit gebundenes Bipyridyl, Bichinolyl und Bithienyl.

6. Elektronisches, optoelektronisches oder elektrolumineszentes Bauelement, das mindestens eine Verbindung nach einem der Ansprüche 1 bis 5 enthält.

## Claims

1. Use of an N-heterocyclic compound according to general formulas A or B 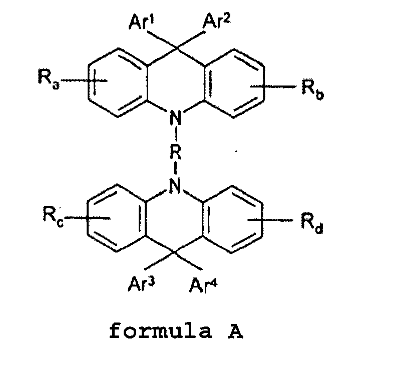 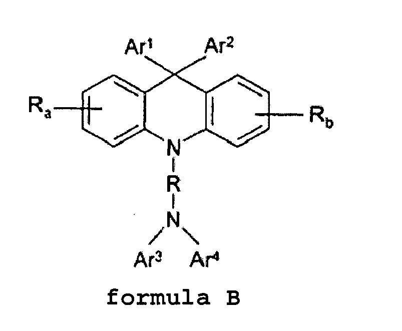 in which:
Ar¹, Ar², Ar³ and Ar⁴, independently of one another, are substituted or unsubstituted, arbitrary aromatic or heteroaromatic residues;
Rₐ, R_{b}, R_{c} and R_{d}, independently of one another, are hydrogen, halogen, alkyl, aryl within the meaning as described for Ar¹-Ar⁴, anellated carbocycle, anellated heterocycle, alkoxy or aryloxy, wherein Rₐ, R_{b}, R_{c} and R_{d} may also occur in multiple; and
R is a structural element bridging both nitrogen-containing groups and containing a system capable of conjugation, wherein R is selected from 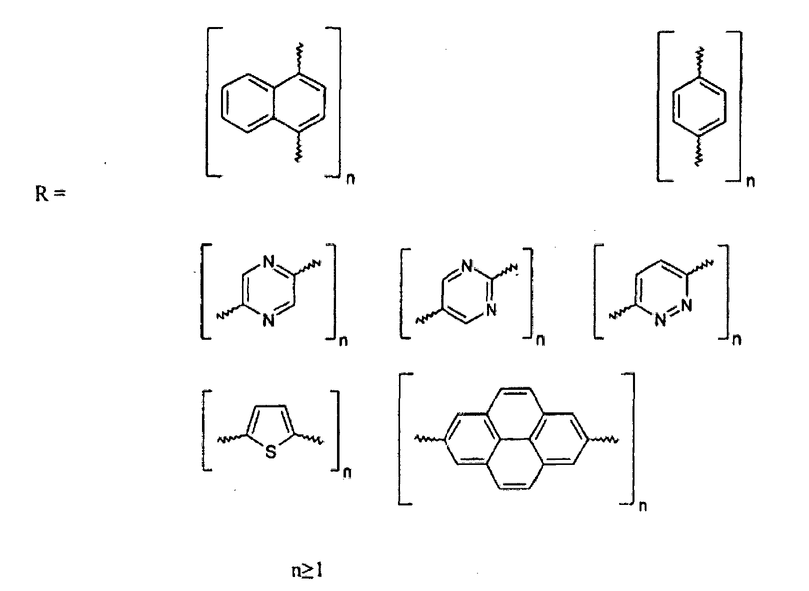 or biphenyl or a dimer, trimer or oligomer of such a structural element, wherein hydroxy-, alkoxy-, cycloalkoxy- and aryloxy-substituted biphenyl is excluded, as a hole conductor in electronic, optoelectronic and electroluminescent components.

2. The use according to Claim 1, **characterised in that** Ar¹, Ar², Ar³ and Ar⁴ are carbocyclic aromatic residues, preferably phenyl, 1-naphthyl, 2-naphthyl, 1-anthryl, 2-anthryl and 9-anthryl.

3. The use according to Claim 1, **characterised in that** Ar¹, Ar², Ar³ and Ar⁴ are homologue carbocyclic aromatic residues, preferably arbitrarily linked biphenyl, binaphthyl, bianthryl and pyrenyl bonded to the acridan unit.

4. The use according to Claim 1, **characterised in that** Ar¹, Ar², Ar³ and Ar⁴ are heteroaromatic residues, preferably 2-pyridyl, 3-pyridyl, 4-pyridyl, 2-quinolyl, 3-quinolyl, 4-quinolyl, 5-quinolyl, 6-quinolyl, 7-quinolyl, 8-quinolyl, 2-thienyl, 3-thienyl and thiazolyl.

5. The use according to Claim 1, **characterised in that** Ar¹, Ar², Ar³ and Ar⁴ are homologue heteroaromatic residues, preferably arbitrarily linked bipyridyl, biquinolyl and bithienyl bonded to the acridan unit.

6. An electronic, optoelectronic or electroluminescent component containing at least one compound according to one of Claims 1 to 5.

## Revendications

1. Utilisation d'un composé N-hétérocyclique selon les formules générales A ou B 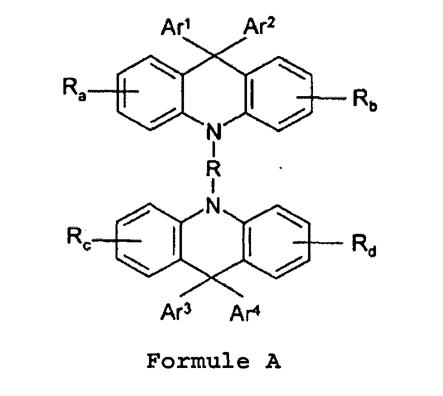 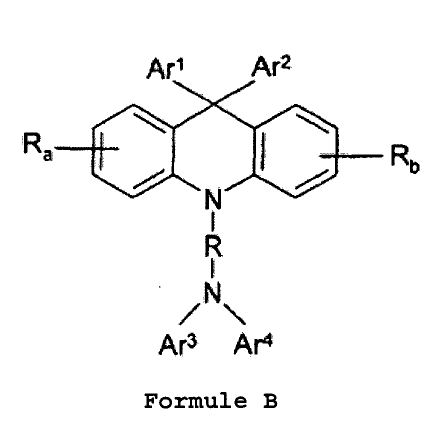 dans lesquelles :
Ar¹, Ar², Ar³ et Ar⁴ représentent, indépendamment l'un de l'autre, des radicaux aromatiques ou hétéroaromatiques qui sont substitués ou non-substitués ;
Rₐ, R_{b}, R_{c} et R_{d} représentent, indépendamment l'un de l'autre, l'hydrogène, un halogène, un alkyle, un aryle avec la signification indiquée pour Ar¹ à Ar⁴, un carbocycle annelé, un hétérocycle annelé, un alkoxy ou un aryloxy, Rₐ, R_{b}, R_{c} et R_{d} pouvant chacun être présent en plusieurs exemplaires ; et
R représente un élément de structure établissant une liaison entre les deux groupes azotés et contenant un système capable de faire partie d'un ensemble conjugué, R étant choisi parmi 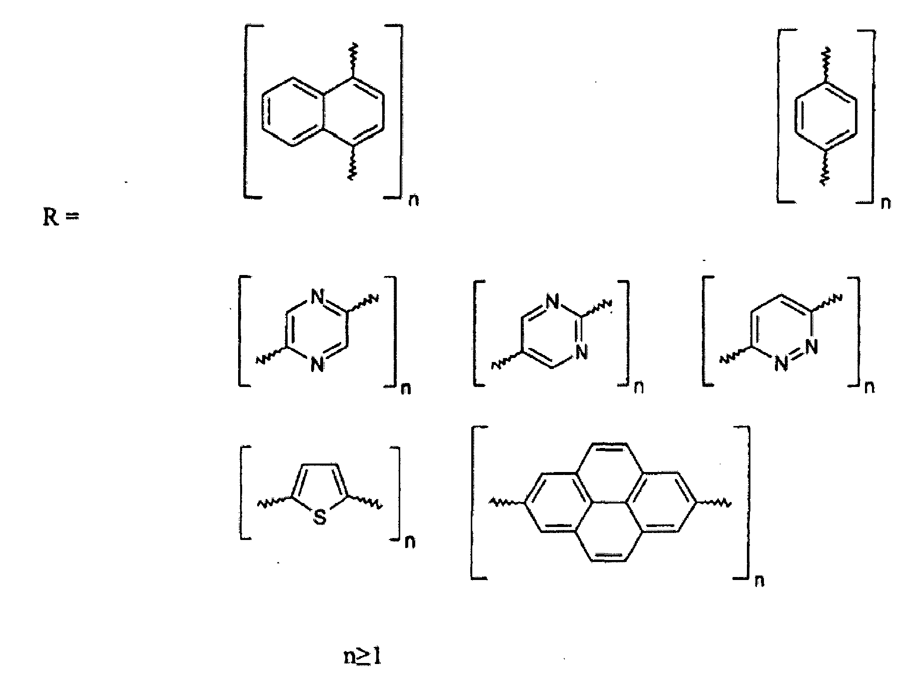 ou un biphényle ou un dimère, trimère ou oligomère d'un tel élément de structure, les biphényles pourvus d'un substituant hydroxyle, alkoxy, cycloalkoxy et aryloxy étant exclus, en tant que matériau conducteur à trous d'électrons dans des composants électroniques, optoélectroniques et électroluminescents.

2. Utilisation selon la revendication 1, **caractérisée en ce que** Ar¹, Ar², Ar³ et Ar⁴ représentent des radicaux aromatiques carbocycliques, s'agissant préférentiellement de phényle, 1-naphtyle, 2-naphtyle, 1-anthryle, 2-anthryle et de 9-anthryle.

3. Utilisation selon la revendication 1, **caractérisée en ce que** Ar¹, Ar², Ar³ et Ar⁴ représentent des homologues de radicaux aromatiques carbocycliques, s'agissant préférentiellement de biphényle, binaphtyle, bianthryle et de pyrényle lesquels sont reliés entre eux d'une quelconque manière et lesquels sont liés à l'unité acridane.

4. Utilisation selon la revendication 1, **caractérisée en ce que** Ar¹, Ar², Ar³ et Ar⁴ sont des radicaux hétéroaromatiques, s'agissant préférentiellement de 2-pyridyle, 3-pyridyle, 4-pyridyle, 2-quinolyle, 3-quinolyle, 4-quinolyle, 5-quinolyle , 6-quinolyle , 7-quinolyle, 8-quinolyle, 2-thiényle, 3-thiényle et de thiazolyle.

5. Utilisation selon la revendication 1, **caractérisée en ce que** Ar¹, Ar², Ar³ et Ar⁴ représentent des homologues de radicaux hétéroaromatiques, s'agissant préférentiellement de bipyridyle, biquinolyle et de bithiényle lesquels sont reliés entre eux d'une quelconque manière et lesquels sont liés à l'unité acridane.

6. Composant électronique, optoélectronique ou électroluminescent contenant au moins un composé selon l'une des revendications 1 à 5.
